(19) Europäisches Patentamt
European Patent Office
Office européen des brevets

(11) Publication number: 0 447 565 A1

(12) **EUROPEAN PATENT APPLICATION published in accordance with Art. 158(3) EPC**

(21) Application number: **90914956.9**

(22) Date of filing: **12.10.90**

(86) International application number: **PCT/JP90/01318**

(87) International publication number: **WO 91/05780 (02.05.91 91/10)**

(51) Int. Cl.[5]: **C07D 319/06**, C09K 19/34, C09K 19/42

(30) Priority: **12.10.89 JP 265664/89**
**20.10.89 JP 273078/89**
**19.12.89 JP 329056/89**

(43) Date of publication of application: **25.09.91 Bulletin 91/39**

(84) Designated Contracting States: **CH DE GB LI**

(71) Applicant: **SEIKO EPSON CORPORATION**
**4-1, Nishi-Shinjuku 2-chome**
**Shinjuku-ku Tokyo 163(JP)**

(72) Inventor: **OBIKAWA, Tsuyoshi**
**3-5, Owa 3-chome**
**Suwa-shi Nagano-ken 392(JP)**
Inventor: **IKUKAWA, Shuji**
**3-5, Owa 3-chome**
**Suwa-shi Nagano-ken 392(JP)**
Inventor: **NAKAYAMA, Jitsuko**
**3-5, Owa 3-chome**
**Suwa-shi Nagano-ken 392(JP)**

(74) Representative: **Caro, William Egerton et al**
**J. MILLER & CO. Lincoln House 296-302 High Holborn**
**London WC1V 7JH(GB)**

(54) **1,3-DIOXANE DERIVATIVE AND LIQUID-CRYSTAL COMPOSITION CONTAINING THE SAME.**

(57) A 1,3-dioxane derivative represented by general formula (I), wherein R is a straight-chain alkyl group having 1 to 10 carbon atoms, A is a single bond or a $-CH_2CH_2-$ group, n is 0 or 1 and the cyclohexane ring and the 1,3-dioxane ring are each in the trans configuration and having very high dielectric anisotropy and small birefringence and, particularly in the form of a liquid-crystal composition containing the same, being-capable of providing a liquid crystal display drivable at a low voltage and having a wide visual angle when applied to a liquid crystal display.

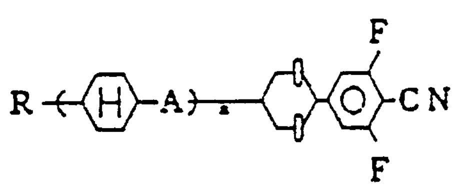

(I)



Rank Xerox (UK) Business Services

## Field Of Technology

The present invention relates to a 1,3-dioxane derivative and liquid crystal compositions containing it used as Np type constituents of large dielectric constant anisotropy ($\Delta\epsilon$), among the constituents that construct nematic liquid crystal compositions used in liquid crystal display apparatus.

## Background Of The Technology

Liquid crystal apparatuses that utilize the electro-optical effect of nematic liquid crystals are being applied in a wider range than priorly. Their display systems include such as dynamic scattering types, guest-host types, twisted nematic types (TN types) and supertwisted nematic types (STN types), and the methods used to drive these display apparatuses include such as static drive systems, time sharing drive systems (dynamic drive systems), active matrix drive systems and 2-frequency drive systems.

The main characterizing features of TN types and STN types that make them superior to emission types of display apparatus such as LED, EL and CRT are such as the following.

(1) Miniaturization and thinning are possible.

(2) Drive voltage is low, and power consumption is very little.

(3) Phasing with LSI is good, and the drive circuit can be simplified.

(4) Because they are light receivers, they are easily seen in direct daylight, and do not cause eyestrain even under long periods of use.

Making the best use of these characterizing features, TN type display apparatuses are being widely applied in displays for such as watches, desk calculators, audio instruments, games, automobile dashboards, cameras, telephone instruments and various types of measuring instruments, and it is estimated that these fields of application will continue to broaden in the future.

Liquid display apparatuses have had their display capacities (number of scanning lines) expanded compared to prior art, together with the expansion in their fields of application. With TN type display apparatuses having time sharing drive systems, the number of scanning lines has a limit on the order of 200, and STN types and TN types with active matrix drive systems are being developed as display systems to replace them. At the present time STN systems are being applied to word processor and personal computer displays and TN types with active matrix drive systems are being applied to liquid crystal color televisions, with the prior main focus being as display apparatuses replacing CRT's.

The various properties indicated below are what are considered to be the indispensable requirements in TN type or STN type liquid crystal display apparatus for such applications, with the understanding that the properties of the liquid crystal materials used in these liquid crystal display apparatuses have various differences in such as their fields of application, display systems and drive methods.

(1) They must have thermal, optical, electrical and chemical stability, without coloration.

(2) They must have a practical temperature range as wide as possible in the vicinity of room temperature.

(3) The threshold value (Vth) of the voltage-light transmission properties (V-Io properties) must be low, and the temperature dependence ($\Delta T$) must be little.

(4) The steepness ($\beta$) of V-Io should be as sharp as possible.

(5) The visual angle dependence ($\alpha$) of V-Io should be as small as possible.

(6) The electro-optical response speed should be fast.

Many liquid crystal compounds and liquid crystal analog compounds are known that satisfy (1) among these various properties, but at present none are known that satisfy the properties from (2) down with a single constituent liquid crystal compound. The actual situation is that liquid crystal compositions containing mixtures of plural nematic liquid crystal mixtures or their analogs are used to satisfy these properties.

After comparing the relation between the properties listed above against the display capacities (number of scanning lines) of the TN type liquid crystal display apparatuses with time sharing drives that are now most widely used, the indispensable requirements for high time sharing drives are these three.

(1) Steepness must be sharp.

(2) Vth temperature dependence must be small.

(3) Visual angle dependence must be little. Considering LSI voltage capacity and power source voltage, there is also this important condition.

(4) The Vth must be low.

These various conditions will be explained next. First steepness ($\beta$) will be represented as Vth and Vsat

$$\beta = \frac{V_{sat}}{V_{th}}$$

Here Vth and Vsat are the voltages when the light transmissivity is respectively 10% and 90%.

Ideally $\beta$ = 1; but it is actually on the order of $\beta$ = 1.3 to 1.5, and it is difficult to develop liquid crystal compounds having a $\beta$ smaller than this value. In the case of the TN type, it is known that $\beta$ decreases as the value of K33/K11 decreases, and it is reported that K33/K11 is small in liquid crystal compounds having pyrimidine structures. Here, K11 and K33 are respectively the splay and bend elastic constants. Vth is expressed ideally for the TN type as follows.

$$V_{th} = \frac{\pi}{d}\sqrt{\frac{K_{11} + (K_{33} - K_{22})/4}{\varepsilon_0 \Delta\varepsilon}}$$

Here d is the thickness of the liquid crystal layer, K11, K22 and K33 are respectively the splay, twist and bend constants of elasticity, and $\epsilon_0$ represents the permitivity of vacuum.

Consequently, to lower Vth, the constant of elasticity should be small and a liquid crystal compound having a large $\Delta\epsilon$ should be added. Also, the temperature dependence ($\Delta$T) of Vth generally shows a tendency for the Vth to lower along with rises in temperature and has the particular characteristic of dropping sharply in the vicinity of N-I point. This is because of the temperature dependence of the constant of elasticity.

Visual angle dependence ($\alpha$) is a particular property of the TN type or the STN type, and its cause is said to lie in the pretilt arising between the liquid crystal and the surface given orientation treatment.

$\alpha$ can be improved by making the thickness of the liquid crystal layer thinner and making the $\Delta$n of the liquid crystal composition smaller.

In this regard, known liquid crystal compounds having a large $\Delta\epsilon$ and a small $\Delta$n that were used for these purposes priorly are shown next.

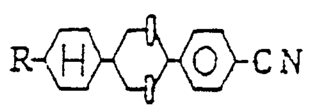

(A)

H-M.Vorbrodt,et.al.,Mol.Cryst.Liq.Cryst.,123,137(1985)

and

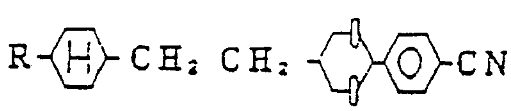

(B)

E.Weinpeter,et.al.,Tetrahedron,44,1609(1988)

These liquid crystal compounds have high N-I points and small $\Delta$n, but they have the defects of a small $\Delta\epsilon$ and poor compatibility with other liquid crystal compounds.

**Disclosure Of The Invention**

The object of the present invention lies in offering a compound having a large $\Delta\epsilon$, a small $\Delta$n, and good compatibility with other liquid crystal compounds.

Another object of the present invention lies in offering a liquid crystal composition capable of high time sharing drive under low voltage, by mixing the said compound with another liquid crystal composition.

The present invention is a 1,3-dioxane derivative represented by the following general formula

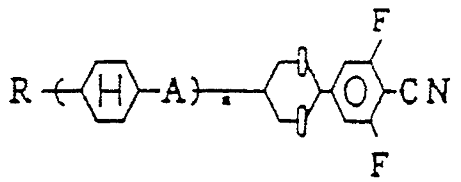

(1)

(where R is a straight chain alkyl group with 1 to 10 carbon atoms, A represents a single bond or a $-CH_2CH_2-$ group, and the cyclohexane ring and 1,3-dioxane ring are respectively trans configurations and n is 0 or 1). It includes the following three types of compounds.

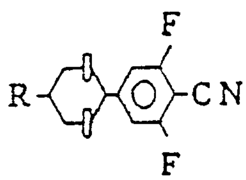

(1 − a)

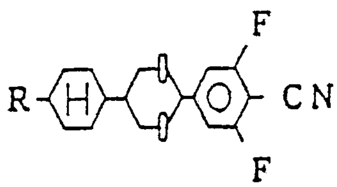

(1 − b)

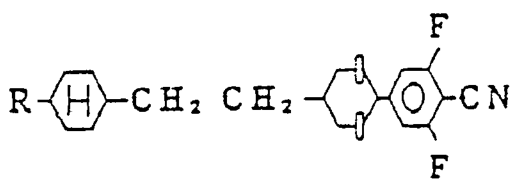

(1 − c)

(where R represents a straight chain alkyl group with 1 to 10 carbon atoms, and the cyclohexane ring and 1,3-dioxane ring are trans configurations.)

Also, the 1,3-dioxane derivative of the present invention is contained as at least one of the ingredients in the liquid crystal composition of the present invention.

Although compound (1-a) of the present invention has no liquid crystal phase, its $\Delta\epsilon$ is very large and its $\Delta n$ is small, so that it is very effective in lowering the Vth of the liquid crystal composition. Compounds (1-b) and (1-c) have nematic phases, and since their $\Delta\epsilon$ are considerably large and their $\Delta n$ are small they can be used as the main ingredients of liquid crystal compositions, making it possible to obtain liquid crystal compositions having low Vth and small $\Delta n$ and that are capable of high time sharing drive.

Also, the compound of the present invention has good compatibility with other liquid crystal compounds, and when done suitably it can be mixed with other liquid crystal compounds in proportions of 1 to 60 wt%, although it is preferred to have a range of 1 to 30 wt% when the object is to mix it with all of the prior liquid crystal compounds.

Further, when mixing the compound of the present invention with other liquid crystal compounds, while the objects will be achieved by using the compound of the present invention as a single ingredient, but it will be more effective to use it mixed as plural ingredients for lowering the melting point in eutectic compositions.

There are no restrictions as to the prior liquid crystal compounds that are capable of being mixed with the compound of the present invention, and liquid crystal compounds of the following systems may be considered for such mixture.

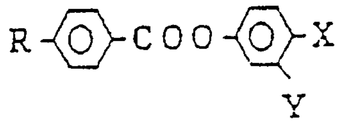

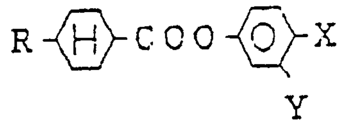

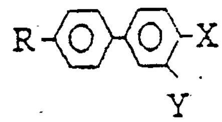

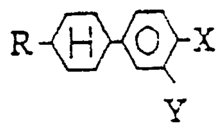

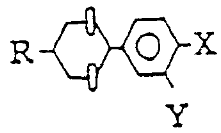

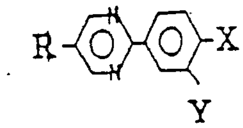

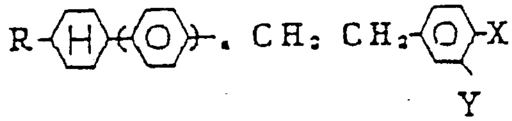

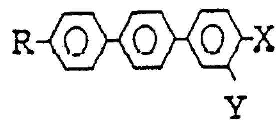

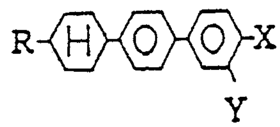

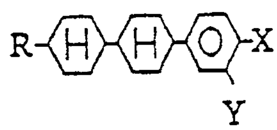

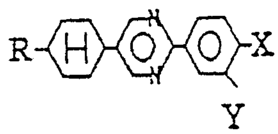

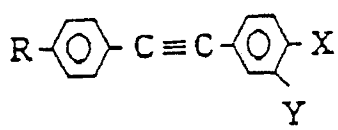

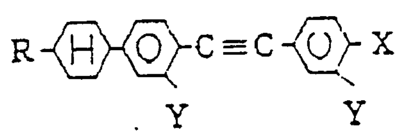

(where, R is a straight chain alkyl group or alkoxy group, X is a straight chain alkyl group, alkoxy group, CN or F, Y is H, F or Cl and n is 0 or 1).

The method of manufacturing the 1,3-dioxane derivative of the present invention will be discussed next.

With the present invention, the compound shown by the general formula

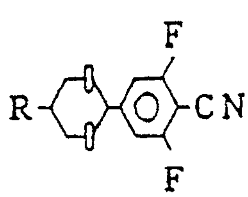

(1 − a)

(where R is a straight chain alkyl group of 1 to 10 carbon atoms, and the 1,3-dioxane ring has a trans configuration) can be manufactured by Schemes 1, 2 and 3 below.

Scheme 1

$R-Br$ (2)

Step 1-1 $CH_2(COOC_2H_5)_2$, $NaOC_2H_5/C_2H_5OH$

$R-CH(COOC_2H_5)_2$ (3)

Step 1-2 $LiAlH_4/THF$

$R-CH(CH_2OH)_2$ (4)

In Scheme 1, the starting material is bromoalkane, and 2-alkylpropane-1,3-diol is obtained by a two stage reaction. In Scheme 2, the starting material is 6-dibromoaniline and 4-bromo-3,5-difluorobenzaldehyde is obtained by a four stage reaction. In Scheme 3, trans-2(4'-cyano-3',5'-difluorophenyl)-5-alkyl-1,3-dioxane (1-a) of the present invention is obtained by a two stage reaction from 2-alkylpropane-1,3-diol and 4-bromo-3,5-difluorobenzaldehyde.

The method of manufacturing the compound (1-a) will next be explained overall in the order of the steps.

Step 1-1. Diethyl alkylmalonate (3) is obtained by an alkylation reaction of bromoalkane (2) and diethyl malonate in anhydrous ethanol under presence of sodium ethoxide ($NaOC_2H_5$).

Step 1-2. 2-alkylpropane-1,3-diol (4) is obtained by reducing compound (3) using lithium aluminum hydride ($LiAlH_4$) in anhydrous tetrahydrofuran (THF).

Scheme 2

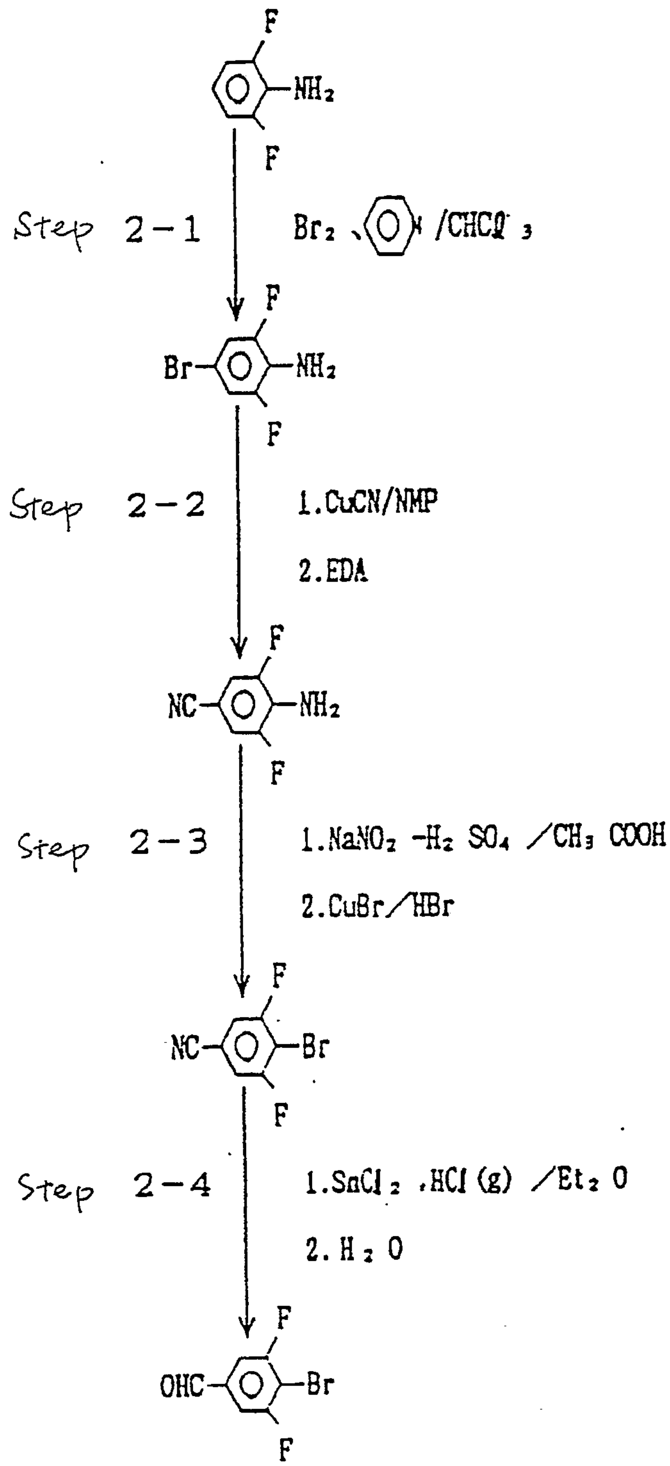


(5)

(6)

(7)

(8)

(9)

Step 2-1. 4-bromo-2,6-difluoroaniline (6) is obtained by bromination of 2,6-difluoroaniline (5) using bromine in chloroform under presence of anhydrous pyridine.

Step 2-2. 4-amino-3,5-difluorobenzonitrile (7) is obtained by cyanating compound (6) using copper cyanide (I) (CuCN) in N-methyl-2-pyrolidinone (NMP), and decomposing the nitrile copper complex of nitrile obtained using ethylene diamine (EDA).

Step 2-3. 4-bromo-3,5-difluorobenzonitrile (8) is obtained by diazotization of compound (7) using nitrocyl hydrogen sulfate ($HCO_4$ -$ONO_2$) prepared from sodium nitrite ($NaNO_2$) and concentrated sulfuric acid in glacial acetic acid, and by bromination of this diazonium salt using copper bromide (I) in hydrobromic acid.

Step 2-4. 4-bromo-3,5-difluorobenzaldehyde (9) is obtained by reducing compound (8) by using tin chloride (II) in diethyl ether saturated with hydrogen chloride gas, and decomposing the tin complex of the imine thus formed with water.

Scheme 3

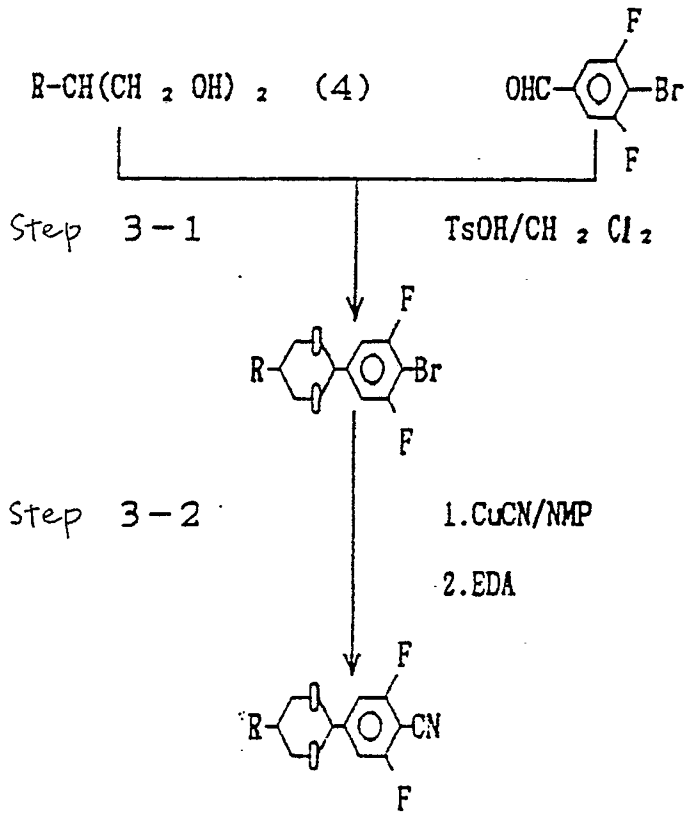


(9)

(10)

(1-a)

(where R is a straight chain alkyl group of 1 to 10 carbon atoms, and the 1,3-dioxane ring is a trans configuration.)

Step 3-1. 2-(4'-bromo-3',5'-difluorophenyl)-2-alkyl-1,3-dioxane (10) is obtained by a dehydration reaction of compound (4) and compound (9) with p-Toluenesulfomic acid as a catalyst in dichloromethane.

Step 3-2. The compound of the present invention trans-2-(4'-cyano-3',5'-difluorophenyl)-2-alkyl-1,3-dioxane is obtained by cyanotizing compound (10) using CuCN in NMP and decomposing the copper complex of nitrile thus produced using EDA.

With the present invention the compound shown by the general formula

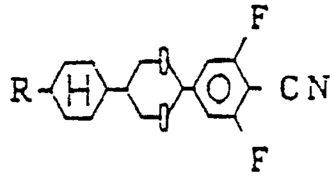

(1-b)

(where, R shows a straight chain alkyl group with a carbon number from 1 to 10, and the cyclohexane ring and the 1,3-dioxane ring has a trans configuration can be made for example by the process shown in Schemes 2 and 4.

In Scheme 4, 2-(trans-4'-alkylcyclohexyl)propane-1,3-diol is obtained by a 3-stage reaction with 4-alkylcyclohexanol as the starting material. In Scheme 2, 4-bromo-3,5-difluorobenzaldehyde is obtained by a 4-stage reaction with 2,6-difluoroaniline as the starting material. In Scheme 5, the liquid crystal compound trans-2-(4'-cyano-3',5'-difluorophenyl)-5-(trans-4'-alkylcyclohexyl)-1,3-dioxane (1-b) of the present invention is obtained by a 2-stage reaction from 2-(trans-4'-alkylcyclohexyl)propane-1,3-diol and 4-bromo-3,5-difluorobenzaldehyde.

A summary of the method of making compound (1-b) following the step sequence will be explained next. Steps 2-1 through 2-4 were already explained in the method of making compound (1-a) and so will be abbreviated here. Scheme 4

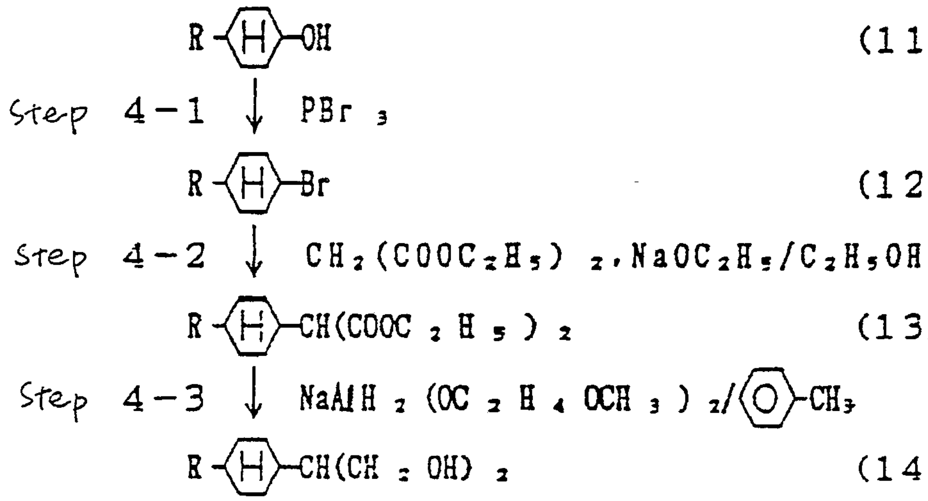


(11)

(12)

(13)

(14)

(where, R shows a straight chain alkyl group of carbon number 1 to 10, and the cyclohexane ring is a mixture of Cis and trans compound.)

Step 4-1. 4-alkylcyclohexanol (11) is brominated using phosphorus tribromide to obtain 4-alkylbromocyclohexane (12).

Step 4-2. Compound (12) and diethyl malonate are given an alkylation reaction using $NaOC_2H_5$ in ethanol anhydride to obtain diethyl 4-alkylcyclohexylmalonate (13).

Step 4-3. Compound (13) is reduced using hydrogenated bis(methoxyethoxy)aluminum sodium hydride in toluene to obtain 2-(4'-alkylcyclohexyl)propane-1,3-diol (14).

Scheme 5

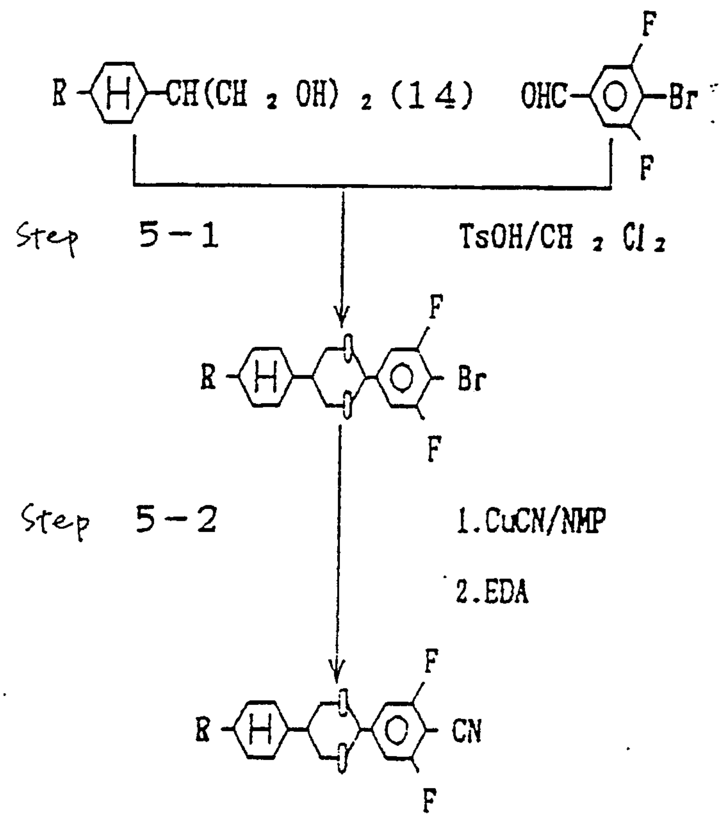


(9)

(15)

(1-a)

(where, R shows a straight chain alkyl group of carbon number 1 to 10, and the cyclohexane ring and 1,3-dioxane ring have trans configuration.)

Step 5-1. Compound (14) and compound (9) are given a dehydration reaction with TsOH as a catalyst in dichloromethane in the same manner as in Step 3 to obtain 2-(4'-bromo-3',5'-difluorophenyl)-5-(4'-alkylcyclohexyl)-1,3-dioxane (15).

Step 5-2. Compound (15) is cyanated using CuCN in NMP in the same manner as in Step 3-2, and the nitrile copper complex of nitrile formed id decomposed with EDA to obtain trans-2-(4'-cyano-3',5'-difluorophenyl)-5-(trans-4'-alkylcyclohexyl)-1,3-dioxane (1-b) of the present invention.

Again, with the present invention, the compound shown by the general formula

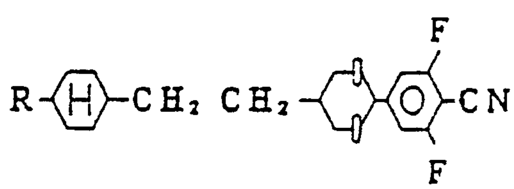

(1-c)

(where, R shows a straight chain alkyl group of carbon number 1 to 10, and the cyclohexane ring and 1,3-dioxane ring has a trans configuration can be made for example by the next process as shown in Schemes 2, 6 and 7.

Scheme 2 was explained with compound (1-a) and so may be abbreviated. In Scheme 6, 2-[2'-(trans-4''-alkylcyclohexyl)ethyl]propane-1,3-diol is obtained by an 8-stage reaction with trans-4-alkylcyclohexanecarboxylic acid as the starting material. In Scheme 7, the trans-2-(4'-cyano-3',5'-difluorophenyl)-5-[2'-(trans-4''-alkylcyclohexyl)ethyl]-1,3-dioxane (1-c) of the present invention is obtained by a 2-stage reaction from 2-(2'-(trans-4''-alkylcyclohexyl)ethyl]propane-1,3-diol and 4-bromo-3,5-difluorobenzaldehyde.

A summary of the method of making compound (1-c) following the order of steps will be explained below. Steps 2-1 to 2-4 were already explained in the method of making compound (1-a) and so will be abbreviated.

Scheme 6

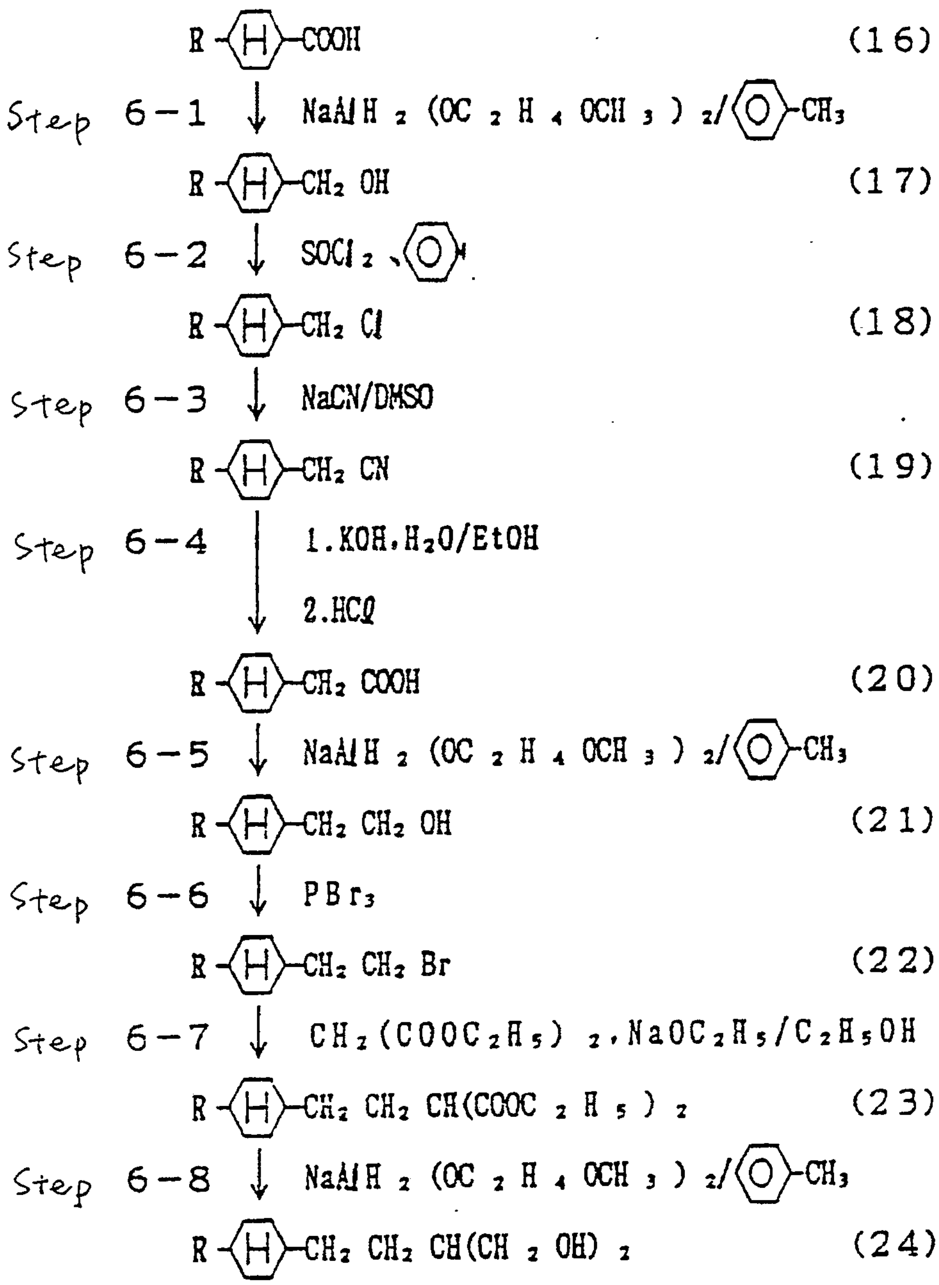


(16)

(17)

(18)

(19)

(20)

(21)

(22)

(23)

(24)

(where, R shows a straight chain alkyl group of carbon number 1 to 10, and the cyclohexane ring has a trans configuration.)

Scheme 7

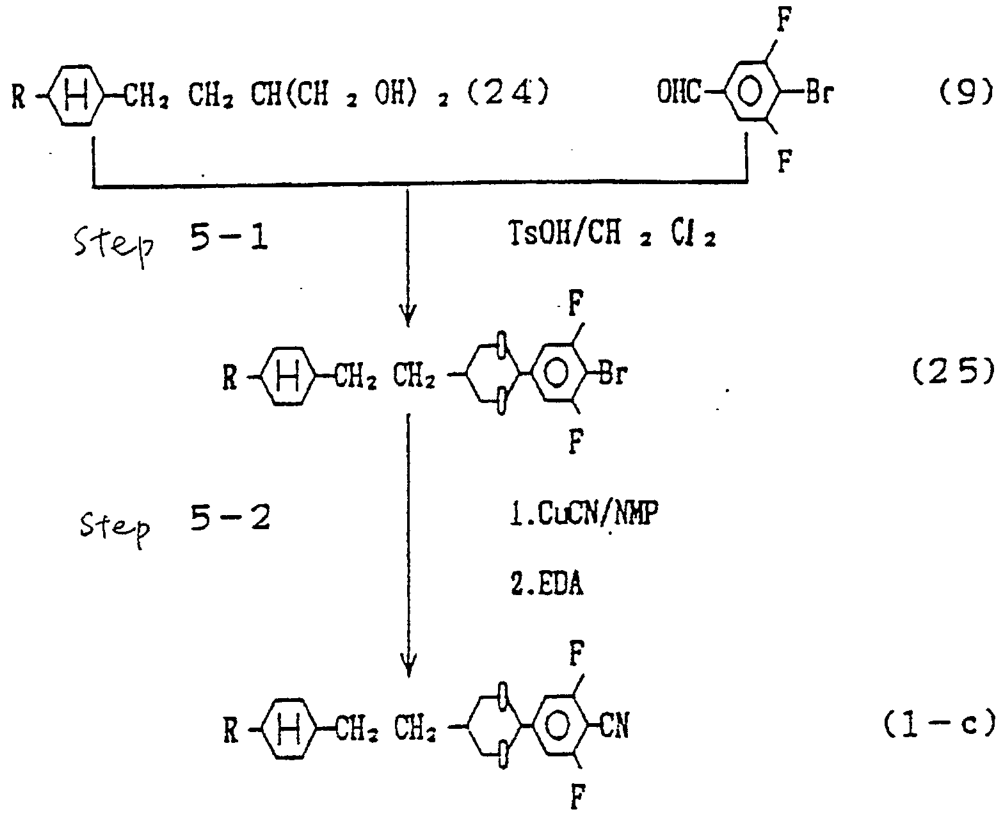


(where, R shows a straight chain alkyl group of carbon number 1 to 10, and the cyclohexane ring and 1,3-dioxane ring have trans configuration.)

Step 6-1. Trans-4-alkylcyclohexanecarboxylic acid (16) is reduced using $NaAlH_2(OC_2H_4OCH_3)_2$ in toluene to obtain trans-4-alkylcyclohexylmethanol (17).

Step 6-2. Compound (17) is chlorinated using thionyl chloride under presence of pyridine to obtain trans-4-alkylcyclohexylchloromethane (18).

Step 6-3. Compound (18) is cyanated using sodium cyanide in dimethyl sulfoxide (DMSO) to obtain trans-4-alkylcyclohexylacetonitrile (19).

Step 6-4. Compound (19) is hydrolyzed using a aqueous KOH solution in ethanol and neutralized in hydrochloric acid to obtain trans-4-alkylcyclohexylacetic acid (20).

Step 6-5. Compound (20) is reduced using $NaAlH_2(OC_2H_4OCH_3)_2$ in toluene in the same manner as in Step. 6-1 to obtain 2-(trans-4'-alkylcyclohexyl)ethane-1-ole (21).

Step 6-6. Compound (21) is brominated using $PBr_3$ to obtain 2-(trans-4'-alkylcyclohexyl)-1-bromoethane (22).

Step 6-7. Compound (22) and diethyl malonate are given an alkylation reaction using $NaOC_2H_5$ in anhydrous ethanol in the same manner as in Step 1-1 to obtain diethyl 2-(trans-4'-alkylcyclohexyl)-ethylmalonate (23).

Step 6-8. Compound (23) is reduced using $NaAlH_2(OC_2H_4OCH_3)_2$ in toluene in the same manner as in Step 4-3 to obtain 2-[2'-(trans-4''-alkylcyclohexyl)ethyl]propane-1,3-diol (24).

Step 7-1. Compound (24) and compound (9) are given dehydration reaction with TsOH as a catalyst in dichloromethane in the same manner as in Step 3-2 to obtain 2-(4'-bromo-3',5'-difluorophenyl)5[2'-(trans-4''-alkylcyclohexyl)ethyl]-1,3-dioxane (25).

Step 7-2. Compound (25) is cyanated using CuCN in NMP in the same manner as in Step 3-2 and the nitrile copper complex formed is decomposed using EDA to obtain trans-2(4'-cyano-3',5'-difluorophenyl)-5-[2'-(trans-4''-alkylcyclohexyl)ethyl]1,3-dioxane (1-c) of the present invention.

## Best Mode For Working The Invention

The present invention will next be explained in further detail by examples and application examples.

Example 1

Method of making 2-pentylpropane-1,3-diol (Scheme 1).

Step 1-1. 23 g (1.0 mol) of Na was dissolved in 500 $cm^3$ of anhydrous ethanol, additions were made of 160 g (1.0 mol) of diethyl malonate and then 151 g (1.0 mol) of bromopentane, and it was refluxed for 7 hours on a hot water bath. The NaBr formed was filtered, the ethanol in the filtrate was removed, and water was added to the residue, it was extracted with chloroform, and washed with water. The chloroform was removed, and the remaining oily substance was distilled under reduced pressure (b. p. 130°C/3 mmHg) to obtain 159 g (0.69 mol) of diethyl pentylmalonate.

Step 1-2. 34 g (0.9 mol) of $LiAlH_4$ was dispersed in 690 $cm^3$ of anhydrous THF, and 159 g (0.69 mol) of diethyl pentyimalonate was added dropwise under stirring. After the dropping was completed, it was refluxed for 2 hours in a hot water bath, and after cooling to room temperature, the excess LiAlH4 was decomposed by dropwise addition of THF including 10% water. 500 $cm^3$ of concentrated hydrochloric acid was added, it was extracted with chloroform and washed in 10% HCl and water. The chloroform was removed, and the remaining oily substance was distilled under reduced pressure (b. p. 110°C/4 mmHg) to obtain 76 g (0.52 mol) of 2-pentylpropane-1,3-diol.

Example 2

Method of making 4-bromo-3,5-difluorobenzaldehyde (Scheme 2).

Step 2-1. 252 g (1.95 mol) of 2,6-difluoroaniline and 170 g (2.15 mol) of anhydrous pyridine were dissolved in 780 $cm^3$ of chloroform, and a solution of 328 g (2.05 mol) of bromine dissolved in 390 $cm^3$ of chloroform was added dropwise for 2 hours on an ice water bath while stirring, after which it was stirred for 2 hours at room temperature. After washing the reaction solution with water, the chloroform was removed, the residue was distilled under reduced pressure (b. p. 100°C/30 mmHg), and the crystals obtained were recrystallized from hexane to obtain 340 g (1.63 mol) of 4-bromo-2,6-difluoroaniline.

Step 2-2. 340 g (1.63 mol) of 4-bromo-2,6-difluoroaniline, 223 g (2.5 mol) of CuCN and 800 $cm^3$ of NMP were refluxed for 1.5 hours on a mantle heater. The reaction solution was cooled to room temperature, and after adding 300 $cm^3$ of EDA, it was poured into 2,000 $cm^3$ of water, extracted with hexane, and washed with an aqueous EDA solution and ice. The hexane was removed, and the residue was distilled under reduced pressure (b. p. 143°C/6 mmHg) to obtain 108 g (0.7 mol) of 4-amino-3,5-difluorobenzonitrile.

Step 2-3. While stirring 420 $cm^3$ of concentrated $H_2SO_4$ on an ice water bath, 54 g (0.78 mol) of pulverized $NaNO_2$ was added at a rate that maintained a temperature under 40°C, and after the addition it was stirred in a hot water bath at 50°C until the crystals dissolved completely. While stirring this solution on an ice water bath, 700 $cm^3$ of glacial acetic acid was added dropwise, then 108 g (0.7 mol) of 4-amino-3,5-difluorobenzonitrile was added at a rate maintaining the temperature at 20-25°C, and after the addition was completed it was stirred at this temperature until the crystals were completely dissolved to prepare a diazonium salt solution. A solution with 143 g (1.0 mol) of CuBr dissolved in 420 $cm^3$ of 47% HBr was stirred in an ice water bath while the previously prepared diazonium salt solution was added dropwise for 2 hours, and after the dropping was completed, it was stirred on an ice water bath for 1 hour and at room temperature overnight. The crystals formed were filtered, and after washing the crystals in glacial acetic acid, they were recrystallized from a mixture of acetone and methanol to obtain 98 g (0.44 mol) of 4-bromo-3,5-difluorobenzonitrile.

Step 2-4. 167 g (0.88 mol) of $SnCl_2$ was added to 880 $cm^3$ of diethyl ether, and dried HCl gas was absorbed to saturation at room temperature. 98 g (0.44 mol) of 4-bromo-3,5-difluorobenzonitrile was added all at once to this mixture, and after stirring for 1 hour, it was let stand overnight. The reactant was poured into 1,000 $cm^3$ of water, and ether was distilled on a hot water bath at 50°C. The residue was extracted by adding chloroform, and after washing in water the chloroform was removed. The residue was distilled under reduced pressure (b. p. 96°C/73 mmHg) to obtain 88 g (0.40 mol) of 4-bromo-3,5-difluorobenzaldehyde.

Example 3

Method of making trans-2-(4'cyano-3',5'-difluorophenyl)-5-pentyl 1,3-dioxane (Scheme 3).

Step 3-1. 10.8 g (0.074 mol) of 2-pentylpropane-1,3-diol, 16.3 g of 4-bromo-3,5-difluorobenzaldehyde and 0.7 g of TsOH were dissolved in 74 $cm^3$ of dichloromethane, and was refluxed for 3 hours on a hot

water bath with a Dean-Stark trap applied to remove the water formed. The reactant was washed in water, the dichloromethane was removed, and the residue was recrystallized from a mixture of acetone and methanol to obtain 9.1 g (0.026 mol) of 2-(4'-bromo-3',5'-difluorophenyl)-5-pentyl-1,3-dioxane.

Step 3-2. 9.1 g (0.026 mol) of 2-(4'-bromo-3',5'-difluorophenyl)-5-pentyl-1,3-dioxane, 3.5 g (0.0039 mol) of CuCN and 78 $cm^3$ of NMP were refluxed for 2 hours on a mantle heater. The reactant was cooled to room temperature, and after adding 10 $cm^3$ of EDA, it was poured in ice water, extracted with hexane and washed with an aqueous EDA solution and water. The hexane was removed, the residue was treated in a silica gel column with chloroform as solvent, and the chloroform was removed. The residue was recrystallized from a mixture of methanol and water to obtain 1.7 g (0.006 mol) of trans-2-(4'-cyano-3',5'-difluorophenyl)-5-pentyl-1,3-dioxane. The phase transition temperature of this compound as measured using DSC was as follows.

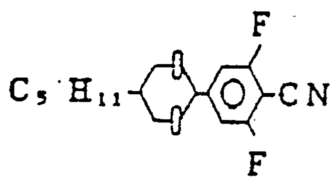

25. 7℃

C ⟶ I

(where, C indicates crystal, and I shows isotropic liquid).

The following compounds were made by the same method of manufacture as Example 3.

Trans-2-(4'-cyano-3',5'-difluorophenyl)-5-methyl-1,3-dioxane
Trans-2-(4'-cyano-3',5'-difluorophenyl)-5-ethyl-1,3-dioxane
Trans-2-(4'-cyano-3',5'-difluorophenyl)-5-propyl-1,3-dioxane
Trans-2-(4'-cyano-3',5'-difluorophenyl)-5-butyl-1,3-dioxane
Trans-2-(4'-cyano-3',5'-difluorophenyl)-5-hexyl-1,3-dioxane
Trans-2-(4'-cyano-3',5'-difluorophenyl)-5-heptyl-1,3-dioxane
Trans-2-(4'-cyano-3',5'-difluorophenyl)-5-octyl-1,3-dioxane
Trans-2-(4'cyano-3',5'-difluorophenyl)-5-nonyl-1,3-dioxane
Trans-2-(4'cyano-3',5'-difluorophenyl)-5-decyl-1,3-dioxane

Example 4

Method of making 2-(4'-propylcyclohexyl)propane-1,3-diol (trans-cis form mixture) (Scheme 4).

Step 4-1. 142 g (1.0 mol) of 4-propylcyclohexanol (trans-cis form mixture) was given drop addition of 136 g (0.5 mol) of $PBr_3$ while stirring at room temperature, and teated to about 80° C. The reactant was stirred for 1 hour on a 70° C hot water bath, then cooled to room temperature and poured into ice water to decompose the excess $PBr_3$. The oily layer was taken off, and the water layer was extracted with chloroform, combined with the oily layer and washed with water. The chloroform was removed, and the remaining oily substance was distilled under reduced pressure (b. p. 97° C/14 mmHg) to obtain 180 g (0.88 mol) of 4-propylbromocyclohexane (trans-cis form mixture).

Step 4-2. 31 g (1.35 mol) of sodium was dissolved in 660 $cm^3$ of anhydrous ethanol, after which 216 g (1.35 mol) of diethyl malonate and then 180 g (0.88 mol) of 4-propylbromocyclohexane were added in single additions, and it was refluxed for 10 hours on a hot water bath. The reactant was cooled to room temperature, and after filtering the NaBr crystals that had formed, the ethanol in the filtrate was removed. Water was added to the residue, it was extracted with chloroform, and washed with water. The chloroform was removed, and the remaining oily substance was distilled under reduced pressure (b. p. 138° C/1.4 mmHg) to obtain 108 g (0.38 mol) of diethyl 4-propylcyclohexylmalonate (trans-cis form mixture).

Step 4-3. 340 $cm^3$ (1.3 mol) of a 70% $NaAlH_2(OC_2H_4OCH_3)_2$ in toluene with 340 $cm^3$ of anhydrous toluene was stirred at room temperature while adding 57 g (0.2 mol) of diethyl 4-propylcyclohexylmalonate dropwise for 30 minutes, after which it was stirred for 5 hours on a hot water bath at 80-90° C. The reactant

was cooled to room temperature, and 50 $cm^3$ of water and 1,000 $cm^3$ of 15% HCl were dropped while stirring. The oily layer was removed, and the water layer was extracted with toluene and combined with the oily layer and washed with 10% HCl and water. The toluene was removed, and the residue was recrystallized from hexane to obtain 18 g (0.09 mol) of 2-(4'-propylcyclohexyl)propane-1,3-diol (trans-cis form mixture).

Example 5

Method of making trans-2-(4'-cyano-3',5'-difluorophenyl)-5-(trans-4'-propylcyclohexyl)-1,3-dioxane (Scheme 5).

Step 5-1. 6.0 g (0.03 mol) of 2-(4'-propylcyclohexyl)propane-1,3-diol, 6.6 g (0.03 mol) of 4-bromo-3,5-difluorobenzaldehyde and 0.3 g of TsOH were dissolved in 60 $cm^3$ of dichloromethane, and it was refluxed for 3 hours on a hot water bath while removing the water produced with a Dean-Stark trap. After washing the reactant on water, the dichloromethane was removed. The residue was recrystallized from a solvent mixture of acetone and methanol to obtain 7.3 g (0.018 mol) of 2-(4'-bromo-3',5'-difluorophenyl)-5(4'-propylcyclohexyl)-1,3-dioxane (trans-cis form mixture).

Step 5-2. 7.3 g (0.018 mol) of 2-(4'-bromo-3',5'-difluorophenyl)-5-(4'-propylcyclohexyl)-1,3-dioxane, 2.5 g (0.027 mol) of $CuCN_2$ and 54 $cm^3$ of NMP were refluxed for 2 hours with a mantle heater. After cooling the reactant to room temperature, 40 $cm^3$ of EDA was added, it was poured into 50 $cm^3$ of ice water, extracted with chloroform and washed with an aqueous EDA solution and water. The chloroform was removed, and the residue was dissolved in hexane and washed with water to remove the NMP. The hexane was removed, and the residue was treated in a silica gel column with chloroform as the solvent. The chloroform was removed, and the residue was repeatedly recrystallized in a solvent mixture of acetone and methanol until there were no cis forms to obtain 1.4 g (0.004 mol) of trans-2-(4'-cyano-3',5'-difluorophenyl)-5-(trans-4'-propylcyclohexyl)-1,3-dioxane. The phase transition temperature of this compound was measured with DSC as follows.

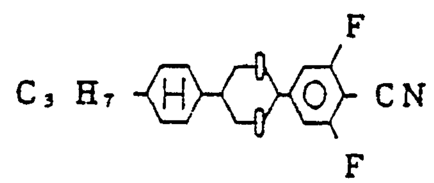

$$C \xrightarrow{72.2^\circ C} N \xrightarrow{115.9^\circ C} I$$

(here, C shows crystals, N shows the nematic phase and I shows isotropic liquid.)

The following compounds were made by the same method of manufacture as in Example 5.

Trans-2-(4'cyano-3',5'-difluorophenyl)-5-(trans-4'-methylcyclohexyl)-1,3-dioxane

Trans-2-(4'-cyano-3',5'-difluorophenyl)-5-(trans-4'-ethylcyclohexyl)-1,3-dioxane

Trans-2-(4'-cyano-3',5'-difluorophenyl)-5-(trans-4'-butylcyclohexyl)-1,3-dioxane

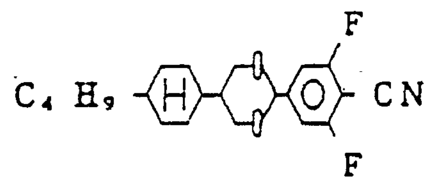

$$C \xrightarrow{58.7^\circ C} N \xrightarrow{117.2^\circ C} I$$

Trans-2-(4'-cyano-3',5'-difluorophenyl)-5-(trans-4'-pentylcyclohexyl)-1,3-dioxane

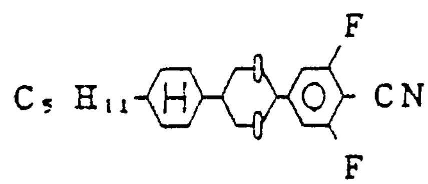

$$C \xrightarrow{65.4^\circ C} N \xrightarrow{126.2^\circ C} I$$

Trans-2-(4'-cyano-3',5'-difluorophenyl)-5-(trans-4'-hexylcyclohexyl)-1,3-dioxane

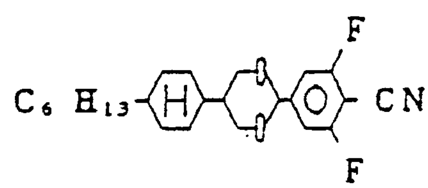

$$C \xrightarrow{77.2^\circ C} N \xrightarrow{119.5^\circ C} I$$

Trans-2-(4'-cyano-3',5'-difluorophenyl)-5-(trans-4'-heptylcyclohexyl)-1,3-dioxane
Trans-2-(4'cyano-3',5'-difluorophenyl)-5-(trans-4'-octylcyclohexyl)-1,3-dioxane
Trans-2-(4'-cyano-3',5'-difluorophenyl)-5-(trans-4'-nonylcyclohexyl)-1,3-dioxane
Trans-2-(4'-cyano-3',5'-difluorophenyl)-5-(trans-4'-decylcyclohexyl)-1,3-dioxane

Example 6

Method of making 2-[2'-(trans-4'-butylcyclohexyl)ethyl]propane-1,3-diol (Scheme 6).

Step 6-1. 55 g (0.30 mol) of trans-4-butylcyclohexane carboxylic acid was dispersed in 200 $cm^3$ of toluene, and while stirring at room temperature, 250 $cm^3$ (0.90 mol) of a 70% $NaAlH_2(OC_2H_4OCH_3)_2$ in toluene was added dropwise at a rate where the toluene refluxed quietly, after which it was stirred for 3 hours on a 90 °C hot water bath. The reactant was cooled to room temperature, 10 $cm^3$ of water and 300 $cm^3$ of 15% HCl were added dropwise while stirring, the oily layer was taken off, the water layer was extracted with toluene and combined with the oily layer, and they were washed with 10% HCl and water. The toluene was removed, and the remaining oily substance was distilled under reduced pressure (b. p. 100 °C/2 mmHg) to obtain 37 g (0.22 mol) of trans-4-butylcyclohexylmethanol.

Step 6-2. 37 g (0.22 mol) of trans-4-butylcyclohexylmethanol and 18 g (0.23 mol) of anhydrous pyridine were given dropwise addition of 32 g (0.27 mol) of $SOCl_2$ in an ice water bath while stirring, and then stirred for 3 hours at 105-110 °C with a mantle heater. The reactant was cooled to room temperature, poured into a beaker containing concentrated HCl and ice, the oily layer was taken off, the water layer was extracted with chloroform, combined with the oily layer and washed with 10% HCl and water. The chloroform was extracted, and the remaining oily substance was distilled under reduced pressure (b. p. 80 °C/3 mmHg) to obtain 38 g (0.20 mol) of trans-4-butylcyclohexylchloromethane.

Step 6-3. 38 g (0.20 mol) of trans-4-butylcyclohexylchloromethane, 12 g (0.24 mol) of NaCN and 40 $cm^3$ of DMSO were heated up to 140 °C with a mantle heater while stirring. The reactant was cooled to room temperature, 1,000 $cm^3$ of water was added, it was extracted with chloroform, and after washing with water the chloroform was extracted. The remaining oily substance was distilled under reduced pressure (b. p. 90 °/3 mmHg) to obtain 34 g (0.19 mol) of trans-4-butylcyclohexylacetonitrile.

Step 6-4. 34 g (0.19 mol) of trans-4-butylcyclohexylacetonitrile, 43 g (0.76 mol) of KOH, 34 $cm^3$ of water and 190 $cm^3$ of ethanol were refluxed on a hot water bath until formation of ammonia gas stopped. The ethanol in the reactant was removed, the residue was dissolved in 100 $cm^3$ of water, it was poured into a beaker containing 100 $cm^3$ of concentrated HCl and 100 g of ice, and the deposited crystals were filtered and washed in water. The crystals were recrystallized from a solvent mixture of methanol and water to obtain 37 g (0.19 mol) of trans-4-butylcyclohexylactetic acid.

Step 6-5. 37 g (0.19 mol) of trans-4-butylcyclohexyl acetic acid was dispersed in 190 $cm^3$ of anhydrous toluene, 160 $cm^3$ (0.57 mol) of 70% of $NaAlH_2(OC_2H_4OCH_3)_2$ in toluene was added dropwise while stirring at a rate where the toluene refluxed quietly, after which it was stirred for 3 hours on a 90 °C hot water bath. The reactant was cooled to room temperature, 40 $cm^3$ of water and 500 $cm^3$ of 15% HCl were added dropwise while stirring, the oily layer was taken off, and the water layer was extracted with toluene, combined with the oily layer and washed with 10% HCl and water. The toluene was removed, and the remaining oily substance was distilled under reduced pressure (b. p. 103 °C/3 mmHg) to obtain 31 g (0.17 mol) of 2-(trans-4'-butylcyclohexyl)ethanol.

Step 6-6. 31 g (0.17 mol)of 2-(trans-4'-butylcyclohexyl)ethanol was given dropwise addition of 23 g (0.09 mol) of $PBr_3$ while stirring at room temperature, after which it was stirred for 1 hour on a 70 °C hot water bath. The reactant was cooled to room temperature, poured into ice water to decompose the excess $PBr_3$, the oily layer was taken off, and the water layer was extracted with chloroform and combined with the oily layer and washed with water. The chloroform was removed, and the remaining oily substance was distilled under reduced pressure (b. p. 99 °C/2 mmHg) to obtain 32 g (0.13 mol) of 2-(trans-4'-butylcyclohexyl)-1-bromoethane.

Step 6-7. 3.0 g (0.13 mol) of sodium was dissolved in 130 $cm^3$ of anhydrous ethanol, 26 g (0.16 mol) of diethyl malonate and 32 g (0.13 mol) of 2-(trans-4'-butylcyclohexyl)-1-bromoethane were added and it was refluxed for 5 hours on a hot water bath. The reactant was cooled to room temperature, the NaBr formed was filtered, and after removing the ethanol in the filtrate, water was added, it was extracted with chloroform and washed with water. The chloroform was removed, and the remaining oily substance was distilled under reduced pressure (b. p. 165 °C/3 mmHg) to obtain 36 g (0.10 mol) of diethyl 2-(trans-4'-buthylcyclohexyl) ethylmalonate

Step 6-8. 170 $cm^3$ (0.60 mol) of 70% of $NaAlH_2(OC_2H_4OCH_3)_2$ in toluene was dissolved in 170 $cm^3$ of anhydrous toluene, and after adding 36 g (0.10 mol) diethyl 2-(trans-4-butylcyclohexyl) ethylmalonate dropwise while stirring, it was stirred for 5 hours on a 80-90 °C hot water bath. The reactant was cooled to room temperature, 30 $cm^3$ of water and 500 $cm^3$ of 15% HCl were added dropwise while stirring, and the oily layer was taken off, the water layer was extracted with toluene, combined with the oily layer and washed in 10% HCl and water. The toluene was removed, and the residue was recrystallized from hexane to obtain 18 g (0.08 mol) of 2-[2'-(trans-4''-butylcyclohexyl)ethyl]propane-1,3-diol.

Example 7

Method of making trans-2(4'-cyano-3',5'-difluorophenyl)-5-[2'-(trans-4'-butylcyclohexyl)ethyl]-1,3 dioxane (Scheme 7).

Step 7-1. 7.3 g (0.03 mol) of 2-[2'-(trans-4'-propylcyclohexyl)ethyl]propane-1,3-diol, 6.6 g (0.03 mol) of 4-bromo-3,5-difluorobenzaldehyde, 0.3 g of TsOH and 60 $cm^3$ of dichloromethane were refluxed for 3 hours in a hot water bath while removing the water formed with a Dean-Stark trap. The reactant was washed with water, the dichloromethane was removed, and the residue was recrystallized from a solvent mixture of acetone and methanol to obtain 7.6 g (0.017 mol) of 2-(4'-bromo-3',5'-difluorophenyl)-5-[2'-(trans-4''-

butylcyclohexyl)ethyl]-1,3 dioxane.

Step 7-2. 7.6 g (0.017 mol) of 2-(4'-bromo-3',5'-difluorophenyl)-5-[2'(trans-4''-butylcyclohexyl)ethyl]-1,3-dioxane, 2.3 g (0.026 mol) of CuCN and 52 $cm^3$ of NMP were refluxed for 2 hours with a mantle heater. The reactant was cooled to room temperature, and after adding 4 $cm^3$ of EDA it was poured into 100 $cm^3$ of ice water, extracted with hexane and washed with an aqueous EDA solution and water. The hexane was removed, and it was treated in a silica gel column with chloroform as the solvent. The chloroform was removed, and the residue was recrystallized from a solvent mixture of acetone and methanol to obtain 2.2 g (0.006 mol) of trans-2-(4'-cyano-3',5'-dibluorophenyl)-5-[2'-(trans-4''-butylcyclohexyl)ethyl]-1,3-dioxane. The phase transition temperature of this compound was measured with DSC as follows.

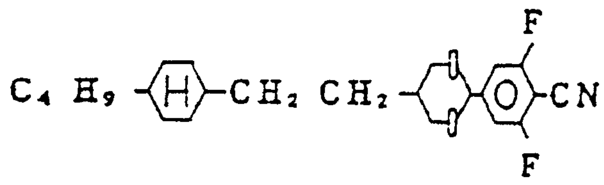

44.8℃ 62.9℃ 96.0℃

C → S → N → I

(where, C shows crystals, S shows the smectic phase, N shows the nematic phase and I shows isotropic liquid.)

The following compounds were made by the same method of manufacture as in Example 7.

Trans-2-(4'-cyano-3',5'-difluorophenyl)-5-[2'-(trans-4'-methylcyclohexyl)ethyl]-1,3'-dioxane

Trans-2-(4'-cyano-3',5'-difluorophenyl)-5-[2'-(trans-4'-ethylcyclohexyl)ethyl]-1,3-dioxane

Trans-2-(4'-cyano-3',5'-difluorophenyl)-5-[2'-(trans-4'-propylcyclohexyl)ethyl]-1,3-dioxane

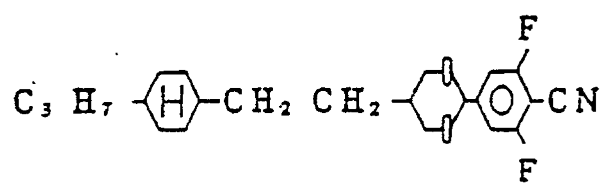

76.8℃ 98.2℃

C → N → I

Trans-2-(4'-cyano-3',5'-difluorophenyl)-5-[2'-(trans-4'-pentylcyclohexyl)ethyl]-1,3-dioxane

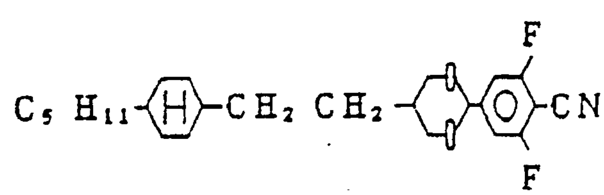

73.0℃ 104.0℃

C → N → I

Trans-2-(4'-cyano-3',5'-difluorophenyl)-5-[2'-(trans-4'-hexylcyclohexyl)ethyl]-1,3-dioxane

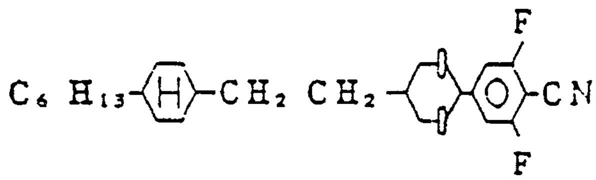

$C \xrightarrow{62.0^{\circ}C} N \xrightarrow{96.8^{\circ}C} I$

Trans-2-(4'-cyano-3',5'-difluorophenyl)-5-[2'-(trans-4'-heptylcyclohexyl)ethyl]-1,3-dioxane
Trans-2-(4'-cyano-3',5'-difluorophenyl)-5-[2'-(trans-4"-octylcyclohexyl)ethyl]-1,3-dioxane
Trans-2-(4'-cyano-3',5'-difluorophenyl)-5-[2'-(trans-4"-nonylcyclohexyl)ethyl]-1,3-dioxane
Trans-2-(4'-cyano-3',5'-difluorophenyl)-5-[2'-(trans-4'-decylcyclohexyl)ethyl]-1,3-dioxane

Application Example 1

The compound of the present invention and compounds (A) and (B) as comparative examples were each mixed separately at 10 wt% with 90 wt% of commercial liquid crystal composition ZLI-1565 (made by Merck Company) to prepare liquid crystal compositions.

Liquid crystal composition [1]

ZLI-1565 90 wt%

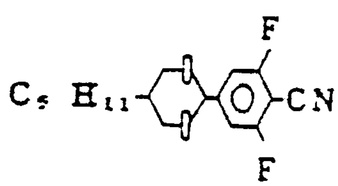

10 wt%

Liquid crystal composition [2]

ZLI-1565 90 wt%

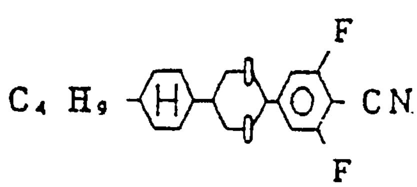

10 wt%

Liquid crystal composition [3]

ZLI-1565 90 wt%

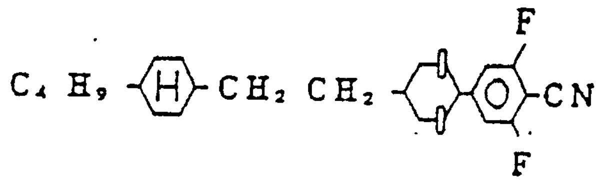

10 wt%

Liquid crystal composition [Comparative Example 1]

ZLI-1565 90 wt%

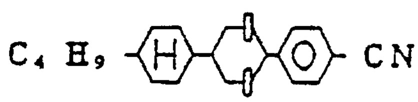

10 wt%

Liquid crystal composition [Comparative Example 2]

ZLI-1565 90 wt%

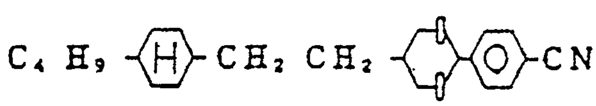

10 wt%

The N-I points and Δn of these liquid crystal compositions were measured. Next, these liquid crystal compositions were enveloped in TN type cells with cell thicknesses of 9 μm, and measurements were made of the threshold voltage (Vth) of the voltage-light transmissivity properties, steepness (β) visual angle dependence (α) at 20°C and the Vth temperature dependence (ΔT) and voltage margin (M) at V -3V and 1/3 duty at 40°C. Here, α,β , ΔT and M were measured by the following formulas.

$$\alpha = \frac{V_{10}\,(T=20^{\circ}\text{、}\ \phi=10^{\circ})}{V_{10}\,(T=20^{\circ}\text{C、}\ \phi=40^{\circ})}$$

$$\beta = \frac{V_{90}\,(T=20^{\circ}\text{C、}\ \phi=10^{\circ})}{V_{90}\,(T=20^{\circ}\text{C、}\ \phi=10^{\circ})}$$

$$\Delta T = V_{10}(T=0^{\circ}\text{C、}\ \phi=10^{\circ}) - V_{10}(T=40^{\circ}\text{、}\ \phi=10^{\circ}) \qquad (V)$$

$$M = \frac{V_{10}(T=40^{\circ}\text{C、}\ \phi=40^{\circ}) - V_{90}(T=0^{\circ}\text{C、}\ \phi=10^{\circ})}{V_{10}(T=40^{\circ}\text{C、}\ \phi=40^{\circ}) + V_{90}(T=0^{\circ}\text{C、}\ \phi=10^{\circ})} \times 100(\%)$$

(where, V10 and V90 are respectively the threshold voltage and saturation voltage, light transmissivity is 10% and 90%, and T represents temperature and $\phi$ the visual angle.)

The above results are shown in Table 1.

Table 1

| Composition | N-I point (°C) | Δn | $V_{th}$ (V) | α | β | ΔT(V) | M(%) |
|---|---|---|---|---|---|---|---|
| [1] | 73.9 | 0.122 | 1.81 | 1.29 | 1.40 | -0.37 | 8.1 |
| [2] | 88.7 | 0.127 | 2.03 | 1.30 | 1.40 | -0.27 | 10.0 |
| [3] | 86.5 | 0.125 | 2.00 | 1.29 | 1.39 | -0.30 | 9.5 |
| | 96.4 | 0.128 | 2.48 | 1.32 | 1.47 | -0.30 | 8.5 |
| | 92.5 | 0.128 | 2.45 | 1.31 | 1.48 | -0.31 | 8.2 |

[Column 1 Heading:] Composition

[Column 2 Heading:] N-I Point (°C)

[Last two lines:

Comparative Example 1

Comparative Example 2

Application Example 2

Liquid crystal compositions [4], [5], [6], [7] and [8] were made from the following components.

Liquid crystal composition [4]

[Numbers:] wt%

L C Composition [4]

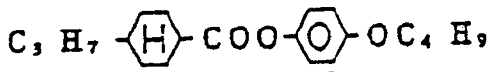
8. 9wt%

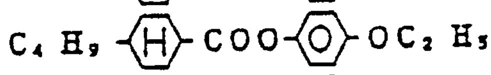
7. 0 〃

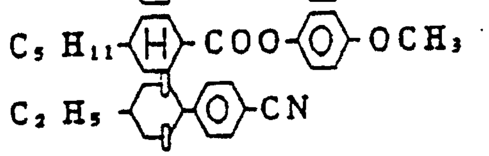
7. 5 〃

10. 4 〃

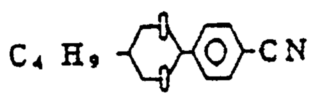
15. 6 〃

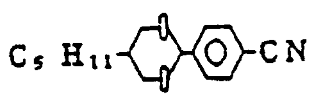
15. 6 〃

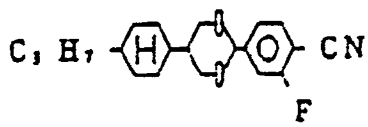
5. 0 〃

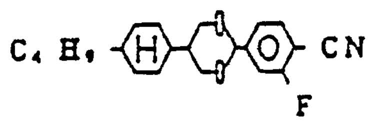
5. 0 〃

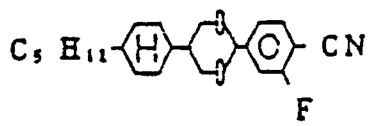
5. 0 〃

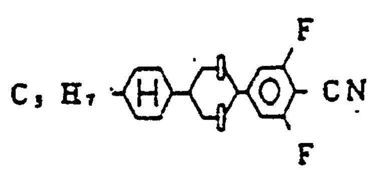
5. 0 〃

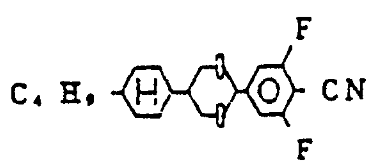
5. 0 〃

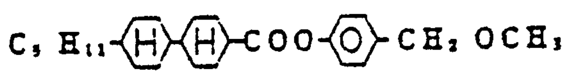
5. 0 〃

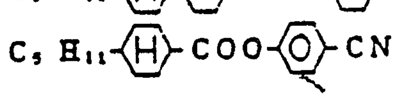
5. 0 〃

Liquid crystal composition [5]

[Numbers:] wt%

(trans form about 80%)

L C Composition [5]

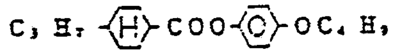 6.8wt%

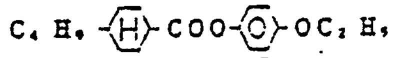 5.4 "

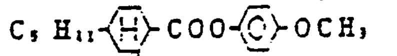 5.8 "

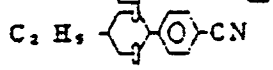 8.0 "

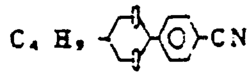 12.0 "

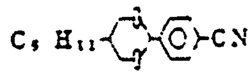 12.0 "

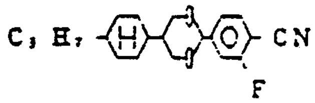 5.0 "

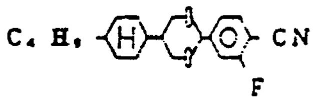 5.0 "

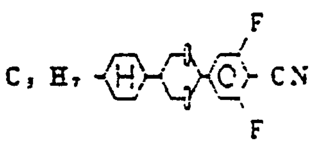 10.0 "

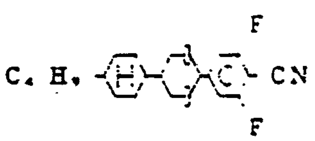 10.0 "

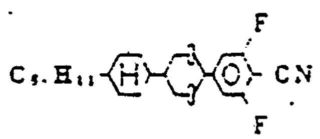 10.0 "

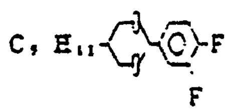 5.0 "

Trans form about 80%)

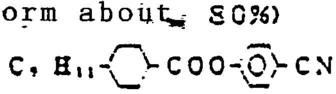 5.0 "

Liquid crystal composition [6]
[Numbers:] wt%
L C Composition [6]

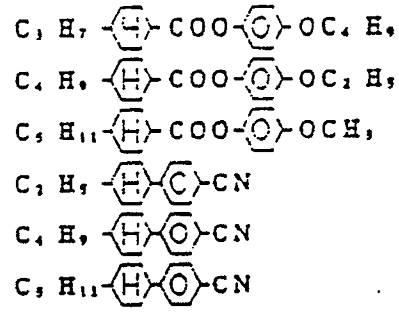

8.9wt%

7.0 〃

7.5 〃

10.4 〃

15.6 〃

15.6 〃

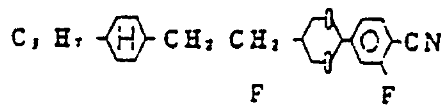

5.0 〃

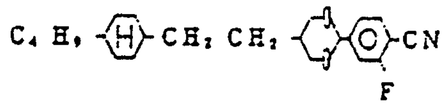

5.0 〃

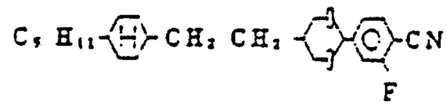

5.0 〃

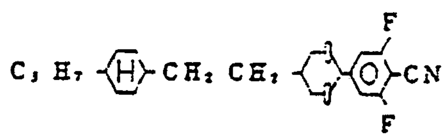

5.0 〃

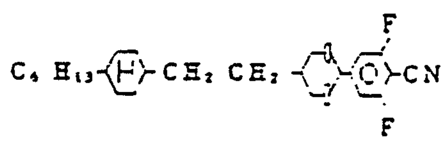

5.0 〃

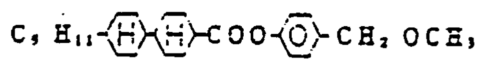

5.0 〃

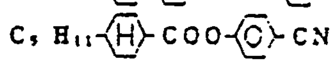

5.0 〃

Liquid crystal composition [7]

[Numbers:] wt%

(trans form about 80%)

液晶組成物〔7〕

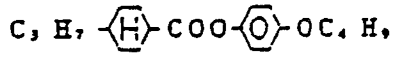 8. 4 wt%

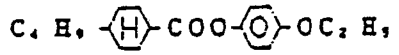 6. 5 〃

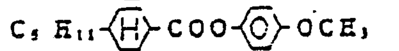 7. 1 〃

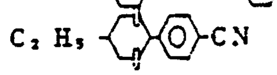 9. 8 〃

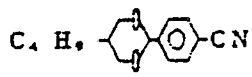 14. 6 〃

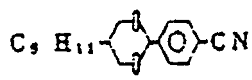 14. 6 〃

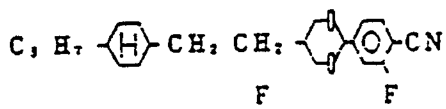 5. 0 〃

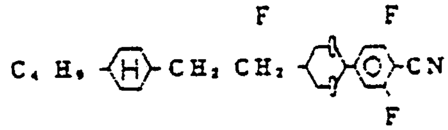 5. 0 〃

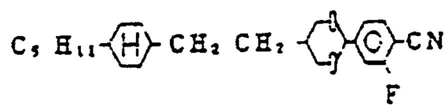 5. 0 〃

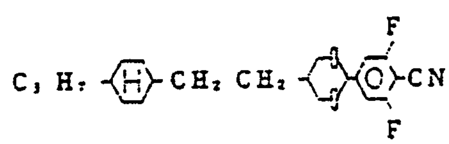 5. 0 〃

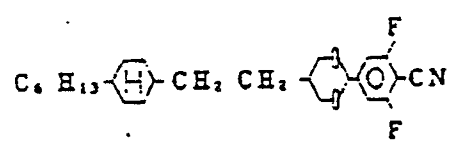 5. 0 〃

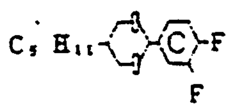 4. 0 〃

Trans form about 80%)

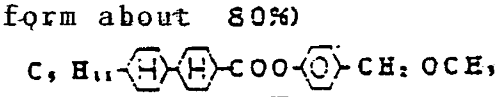 5. 0 〃

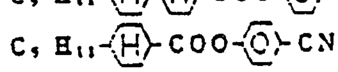 5. 0 〃

Liquid crystal composition [8]

[Numbers:] wt%

(trans form about 80%)

液晶組成物 [8]

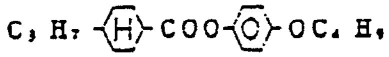 6.8 wt%

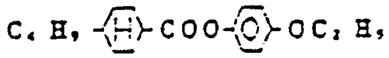 5.4 〃

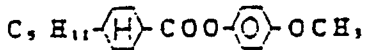 5.8 〃

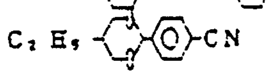 8.0 〃

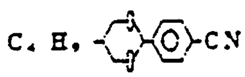 12.0 〃

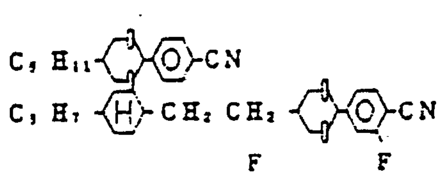 12.0 〃

5.0 〃

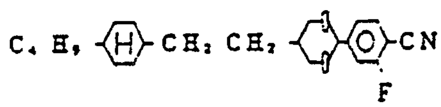 5.0 〃

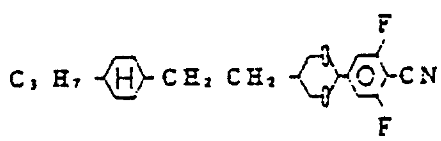 10.0 〃

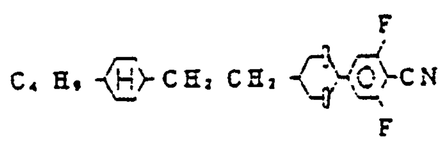 10.0 〃

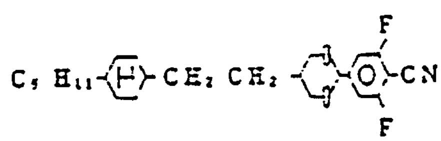 10.0 〃

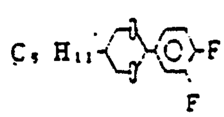 5.0 〃

about form about 80%)

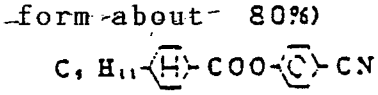 5.0 〃

The N-I point, Δn, Vth, α, β, ΔT and M of these liquid crystal compositions were measured in the same manner as in Application Example 1, and the results are shown in Table 2.

Table 2

| Composition | N-I point (℃) | Δ n | $V_{th}$ (V) | α | β | ΔT(V) | M(%) |
|---|---|---|---|---|---|---|---|
| [4] | 75.1 | 0.108 | 1.15 | 1.28 | 1.47 | 0.343 | 6.3 |
| [5] | 67.8 | 0.102 | 0.91 | 1.28 | 1.48 | 0.423 | 4.5 |
| [6] | 70.4 | 0.105 | 1.14 | 1.28 | 1.45 | 0.391 | 5.5 |
| [7] | 65.5 | 0.100 | 1.07 | 1.26 | 1.48 | 0.440 | 4.0 |
| [8] | 61.7 | 0.099 | 0.87 | 1.29 | 1.49 | 0.376 | 5.3 |

[Column 1 Heading:] Composition

[Column 2 Heading:] N-I Point (°C)

From the results above, it will be understood that the liquid crystal composition of the present invention is capable of high time sharing drive even under low Vth voltage below 1 V.

**Possibility Of Industrial Utilization**

As described above the 1,3 -dioxane derivative of the present invention has a very large Δε, and a small Δn.

Also, it can be confirmed that liquid crystal compositions obtained by mixing the 1,3-dioxane derivative of the present invention with prior liquid crystal compounds have wide visual angles and are capable of high time sharing drive at low voltages.

Consequently, the 1,3-dioxane derivative of the present invention is very useful as a liquid crystal compound or analog compound used in liquid crystal display apparatus of the TN type or STN type.

**Claims**

**1.** 1,3-dioxane derivative characterized in that it is expressed by the general formula

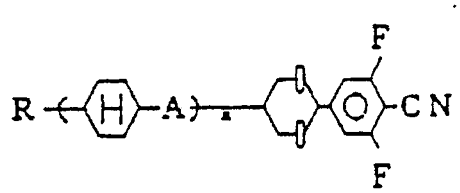

(1)

(where, R is a straight chain alkyl group of carbon number 1 to 10, A is a single bond or $-CH_2CH_2-$

group, n is 0 or 1, and the cyclohexane ring and 1,3-dioxane ring are respectively in trans

2. 1,3-dioxane derivative described in Claim Paragraph 1 characterized in that it is expressed by the general formula

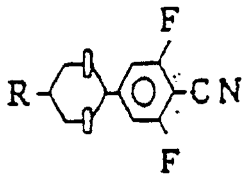

(1 − a)

(where, R is a straight chain alkyl group of carbon number 1 to 10, and the 1,3-dioxane ring is in trans form.)

3. 1,3-dioxane derivative described in Claim Paragraph 1 characterized in that it is expressed by the general formula

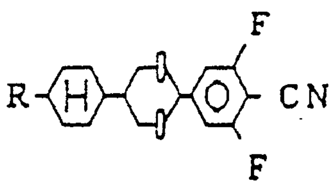

(1 − b)

(where, R is a straight chain alkyl group of carbon number 1 to 10, and the cyclohexane ring and 1,3-dioxane ring are respectively in trans form.)

4. 1,3-dioxane derivative described in Claim Paragraph 1 characterized in that it is expressed by the general formula

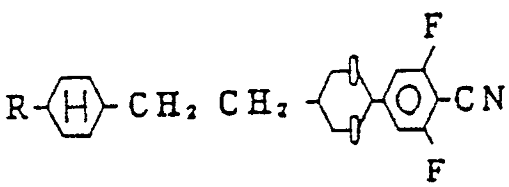

(1 − c)

(where, R is a straight chain alkyl group of carbon number 1 to 10, and the cyclohexane ring and 1,3-dioxane ring are respectively in trans form.)

5. Liquid crystal composition characterized in that it contains as at least one of its ingredients the 1,3-dioxane derivative expressed by the general formula

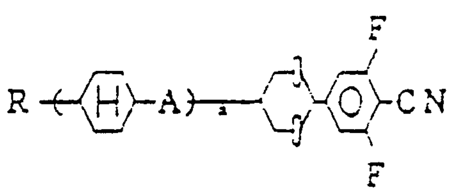

(1)

(where, R is a straight chain alkyl group of carbon number 1 to 10, A is a single bond or $-CH_2CH_2-$ group, n is 0 or 1, and the cyclohexane ring and 1,3-dioxane ring are respectively in trans form.)

6. Liquid crystal composition described in Claim Paragraph 5 characterized in that it contains 1 to 30 wt% of the 1,3-dioxane derivative expressed by the general formula

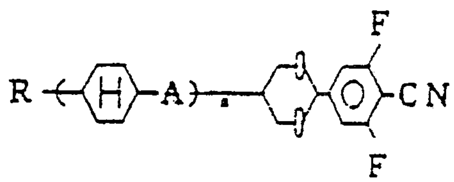

(1)

(where, R is a straight chain alkyl group of carbon number 1 to 10, A is a single bond or $-CH_2CH_2-$ group, n is 0 or 1, and the cyclohexane ring and 1,3-dioxane ring are respectively in trans form.)

# INTERNATIONAL SEARCH REPORT

International Application No PCT/JP90/01318

**I. CLASSIFICATION OF SUBJECT MATTER** (if several classification symbols apply, indicate all) [6]

According to International Patent Classification (IPC) or to both National Classification and IPC

Int. $Cl^5$ C07D319/06, C09K19/34, 19/42

**II. FIELDS SEARCHED**

Minimum Documentation Searched [7]

| Classification System | Classification Symbols |
|---|---|
| IPC | C07D319/00-06, C09K19/34, 42-46 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 62-501216 (Merck Patent GmbH), 14 May 1987 (14. 05. 87), Refer to Examples 10, 11 & DE, 3447359 & WO, A, 8613769 & EP, A, 205503 | 1 - 6 |
| A | JP, A, 56-100886 (Chisso Corp.), 13 August 1981 (13. 08. 81), (Family: none) | 1 - 6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| December 4, 1990 (04. 12. 90) | December 17, 1990 (17. 12. 90) |
| **International Searching Authority** | **Signature of Authorized Officer** |
| Japanese Patent Office | |